# EUROPÄISCHE PATENTSCHRIFT

(11) **EP 2 677 954 B1**
(45) Veröffentlichungstag und Bekanntmachung des Hinweises auf die Patenterteilung: **23.12.2015**
(21) Anmeldenummer: 11810848.9
(22) Anmeldetag: 28.12.2011
(51) Int. Cl.: A61B 19/00, A61C 1/08

(54) **CHIRURGISCHES INSTRUMENT MIT INTEGRIERTER NAVIGATONSKONTROLLE**
SURGICAL INSTRUMENT HAVING INTEGRATED NAVIGATION CONTROL
INSTRUMENT CHIRURGICAL À CONTRÔLE DE NAVIGATION INTÉGRÉ

(30) Priorität: 25.02.2011 DE 102011012460
(43) Veröffentlichungstag der Anmeldung: 01.01.2014
(73) Patentinhaber: Hicat GmbH, 53177 Bonn (DE)
(72) Erfinder: HEY, Joachim, 53639 Königswinter (DE); BREUER, Manfred, 53347 Alfter (DE); HANSSEN, Nils, 53111 Bonn (DE)
(74) Vertreter: Braun-Dullaeus, Karl-Ulrich
(86) Internationale Anmeldenummer: PCT/EP2011/074192
(87) Internationale Veröffentlichungsnummer: WO 2012/113484

(56) Entgegenhaltungen:
- WO-A1-01/35849
- DE-A1- 10 259 250
- US-A- 5 795 294
- US-A- 5 871 445
- US-A1- 2008 208 041

## Beschreibung

Die vorliegende Erfindung betrifft ein System zur Durchführung einer Behandlung eines insbesondere menschlichen oder tierischen Körpers, umfassend ein handhabbares Instrument mit einem den Körper beaufschlagenden und ein Werkzeug tragenden Instrumentenkopf und umfassend eine Kontrolleinheit mit einem Computer, auf dem ein Navigationsprogramm zur Unterstützung der Führung des Instrumentenkopfes realisiert ist, wobei dem Navigationsprogramm den das Operationsfeld beinhaltenden Teil des Körpers repräsentierende Körperdaten, die geplante Behandlung repräsentierende Planungsdaten und die Position und die Ausrichtung des Instrumentenkopfes repräsentierende Instrumentendaten zur Verfügung stehen, wobei ein Ortungsmittel zur Aufnahme der Instrumentendaten vorhanden ist, wobei das Navigationsprogramm die Instrumentendaten ("Ist-Daten") mit den Planungsdaten (Soll-Daten) vergleicht, wobei ein Signalmittel vorgesehen ist, das dem Operateur eine Abweichung der Ist-Daten von den Soll-Daten anzeigt.

Ein solches System für insbesondere Kieferchirurgische Eingriffe, mit dem die Bewegung eines Dentalbohrers im Behandlungsbereich erfasst werden kann, ist aus der DE 102 59 250 A1 bekannt. Dieses bedient sich eines extern positionierten Ortungsmittels in der Art eines Kamerasystems, um die aktuelle Lage des Instrumentenkopfes zu bestimmen. Ein ähnliches System, das sich ebenfalls derart aufwendiger Ortungsmittel bedient, ist in der DE 101 10 093 A1 gezeigt. Ein besonderes Problem der bekannten Systeme ist die Registrierung, also die Transformation der Ist-Daten in das System der Soll-Daten. Um diese Registrierung zu bewerkstelligen, bedienen sich die Systeme besonderer Markerstrukturen, die einerseits an dem Instrument und andererseits am Patient angebracht sind. Die Lage und Orientierung dieser Markerstrukturen im Raum wird über das extern positionierte Ortungsmittel festgestellt und jeweils in die Planungsdaten projiziert. Vermittels der Markerstrukturen kann also die Lage des Patienten und die Lage des Instruments festgestellt und als Ist-Daten in die Planungsdaten (Soll-Daten) einbezogen werden. Abweichungen zwischen Ist- und Soll-Daten werden dem behandelnden Arzt entsprechend der DE 102 59 250 A1 unmittelbar am Instrumentenkopf oder entsprechend der DE 101 10 093 A1 auf einem externen Bildschirm angezeigt.

Auch aus der US 2008/208041 A2 ist ein Behandlungssystem bekannt, welches für den behandelnden Arzt eine Orientierungsmöglichkeit anhand von Markern bereitstellt, die an der Oberfläche des zu behandelnden Körperbereichs angebracht sind.

Diese Systeme sind wegen des komplexen Aufbaus bezüglich der Halterung der Marker problematisch, da die Markerstrukturen aus dem Behandlungsbereich herausragen müssen, um von den externen optischen Ortungsmitteln erfasst werden zu können. Durch die Rücksichtnahme auf die Markerstrukturen ist auch der Arbeitsbereich stark eingeschränkt. Außerdem ist eine aufwendige Kalibrierung und Einrichtung des Systems nicht nur vor Beginn eines Eingriffes notwendig. Auch machen sich während des Eingriffes verändernde Verhältnisse mitunter eine wiederholte Kalibrierung notwendig.

Aus dem Dentalbereich sind neben diesen technisch aufwendigen Systemen auch individuell gefertigte Schablonen bekannt, die einen in seiner Bohrtiefe begrenzten Bohrkanal für den mit der Hand zu führenden Dentalbohrer aufweisen. Der Nachteil dieser Bohrschablonen ist jedoch, dass sie jeweils individuell in aufwendigen Arbeitsgängen gefertigt werden müssen.

Aufgabe der vorliegenden Erfindung ist es nunmehr, ein System zu schaffen, das sich besonders einfach und vor allem ohne Zuhilfenahme externer Ortungsmittel umsetzen lässt und das eine exakte Kontrolle des Instrumentes, mit dem die Planung umgesetzt werden soll, gewährleistet. Auch soll das System flexibel für das Umsetzen von Planungen einsetzbar sein.

Diese Aufgabe wird durch ein System mit den kennzeichnenden Merkmalen des Anspruch 1 gelöst.

Der grundlegende Gedanke der Erfindung liegt darin, das Ortungsmittel unmittelbar am Instrumenten kopf anzubringen, so dass das Instrument nicht durch externe Mittel geortet werden muss, sondern dass sich das Instrument selber "ein Bild" von seiner Umgebung macht, wobei dieses Bild dann mit den Soll-Daten abgeglichen wird.

Zur Aufnahme der Einzelbilder weist dieses unmittelbar am Instrumentenkopf angebrachte Ortungsmittel ein Mittel zur Bildaufnahme auf, das die Aufnahme der Einzelbildern während der Handhabung des Instrumentenkopfes ermöglicht. Aus mindestens zwei Einzelbildern, die gleichzeitig aus verschiedenen Perspektiven oder in schneller Abfolge nach einer Bewegung aufgenommen werden, lassen sich Koordinaten in drei Dimensionen und damit stereoskopische Bilder berechnen. Dabei ist es zum Erhalt einer hohen zeitlichen Sensibilität des Systems vorteilhaft, eine Vielzahl solcher Einzelbilder in schneller zeitlicher Abfolge in der Art einer Filmsequenz aufzunehmen. In einer besonders einfachen und daher vorteilhaften Ausführungsform erzeugt ein solches Bildaufnahmemittel Bilder im optischen Bereich. Es ist aber auch denkbar, Bilder mittels anderer Medien insbesondere mittels Ultraschall aufzunehmen.

Die Einzelbilder müssen nicht zwangsläufig unmittelbar von der Behandlungsstelle stammen. So ist es für die erfindungsgemäße Führung des Instrumentenkopfes im Koordinatensystem der Planungsdaten lediglich notwendig, dass die Einzeibilder ein Körperteil zeigen, das in den Körperdaten repräsentiert ist und das mit der Behandlungsstelle in definiertem Bezug steht. Beispielsweise kann das Bildaufnahmemittel auf den der zu behandelnden Zahnlücke benachbarten Zahn ausgerichtet sein, der seine Entsprechung in den Körperdaten und damit in den Planungsdaten hat. Wesentlich ist dabei auch, dass die Ausrichtung des Bildaufnahmemittels in definiertem unveränderlichen Bezug zum Instrumentenkopf steht, so dass die Einzelbilder entsprechend definierten Bezug zur Behandlungsstelle haben.

Es bietet sich jedoch an, das Aufnahmemittel direkt auf die Behandlungsstelle zu richten und Einzelbilder von der Behandlungsstelle und der unmittelbaren Umgebung aufzunehmen. Durch Abgleich der Einzelbilder mit den Körperdaten kann das Navigationsprogramm die aktuellen Instrumentendaten erzeugen. Wie oben schon ausgeführt, werden die Einzelbilder vorteilhafterweise in schneller Abfolge aufgenommen, so dass sich auch die Bewegung des Instrumentenkopfes als Instrumentendaten in die Körper- und Planungsdaten projizieren lässt. Nach Abgleich der Ist-Daten mit den aus der Planung stammenden Soll-Daten wird dem Operateur die Abweichung von der Planung auf dem Signalmittel anzeigt, so dass er seine Bewegung entsprechend korrigieren kann.

Erfindungsgemäß wird das aus den aufgenommenen Ist-Daten rekonstruierte Bild, das sich dem Aufnahmemittel bietet, in Bezug zu dem die Soll-Daten repräsentierenden Bild, das sich dem Aufnahmemittel bieten sollte, abgeglichen. Aus der Bemühung des Navigationsprogramms, die beiden Bilder in Deckung zu bringen, ergibt sich die auf dem Signalmittel dargestellte Handlungsanweisung an den Operateur. Wenn er dieser folgt, gelangt das Instrument letztendlich in die richtige Position Die Information, in welche Richtung der Operateur sein Instrument in Bezug auf die Planung bewegen muss, ist insbesondere direkt am Ort des Geschehens verfügbar, so dass eine besonders ergonomische Arbeitsweise möglich ist.

Dabei werden die aufgenommenen Bilddaten an einen dem System zugeordneten Rechner übermittelt, der die Strukturen anhand der Oberflächen erkennt und die Lage und Position des Instruments in Bezug auf diese Oberfläche errechnet. Gleichzeitig ist die Position und Lage des geplanten Eingriffs in Bezug zu dieser Oberfläche bekannt. Nun kann der Rechner Steuerbefehle geben, die Position und Lage des Instruments so lange zu korrigieren, bis diese dem geplanten Eingriff entspricht.

Dabei ist es besonders vorteilhaft, wenn das Signalmittel derart am Instrument angebracht ist, dass der Operateur das Signalmittel beobachten kann, während er den Instrumentenkopf im Blickfeld hat. Auf diese Weise werden dem Operateur während der Handhabung des Instrumentes Informationen über die Abweichungen von der Planung gegeben, ohne dass er das manuell geführte Instrument aus dem Blickfeld verliert. Diese Informationen geben dem Operateur die Möglichkeit, eine Korrektur der Instrumentenführung unter der unmittelbaren Kontrolle des Systems vorzunehmen.

Entsprechend dieser Ausführungsform wird die Position und die Lage des Instruments in Bezug auf die Planung am Instrument selber visualisiert, so dass der Operateur nicht auf externe Bildschirme und Displays angewiesen ist, die sich außerhalb des Behandlungsbereiches befinden. Eine solche optische Anzeige am Instrument selber ermöglicht somit eine intuitive Bedienung durch den Operateur, ohne dass dieser seinen Blick vom Ort des Eingriffs abgewendet werden müsste. Die Visualisierung führt sozusagen die Hand des Operateurs in die richtige Richtung. Mit einem erfindungsgemäß ausgestatteten System kann die manuelle Navigation des Instruments, das insbesondere ein Dentalbohrer ist, exakt entsprechend der zuvor erfolgten Behandlungsplanung erfolgen. Das Instrument kann in unmittelbarer Orientierung zu dem Objekt manuell geführt werden, in dessen Nähe der Eingriff statt findet. Das ist möglich, da die Behandlungsplanung in Bezug auf die starre Struktur des Objektes in der Umgebung der Behandlungsstelle erfolgt.

Vorteilhafterweise ist das Blickfeld des Bildaufnahmemittels auf das Operationsfeld und insbesondere auch auf den Instrumentenkopf gerichtet, auf diese Weise ist es möglich, dass das aktuelle Bild des Bildaufnahmemittels (zusätzlich) auf einem für den Operateur einsehbaren Bildschirm angezeigt wird. In einer besonders vorteilhaften Ausführungsform umfasst das Ortungsmittel mindestens zwei Bildaufnahmemittel, um eine exakte Orientierung in allen drei Dimensionen zu ermöglichen. Beispielsweise können die beiden Ortungsmittel so angeordnet sein, dass sie eine zur Erzeugung einer stereoskopischen Aufnahme des Körperteils geeignete Anordnung zueinander haben. Sie können aber auch auf verschiedene Bereiche gerichtet sein, da sich auch aus dem Abgleich zweier unterschiedlicher Bilder die Orientierung im Raum ermitteln lässt. Entscheidens ist lediglich, dass die Anordnung definiert ist und das System auf diese definierte Anordnung geeicht ist.

Um eine exakte Orientierung des Instrumentes gewährleisten zu können, ist es vorteilhaft, dass das Navigationsprogramm zum Abgleich der Einzelbilderoder des Filmes mit den Körperdaten massive Strukturen, wie freigelegten Knochen und/oder Zähne, aus den Körperdaten herausfiltert. Diese Strukturen sind als feste und dauerhafte Bezugspunkte besonders geeignet.

Da Abtastungen ("Scanns") der Oberfläche einer Struktur besonders repräsentative und leicht zu verarbeitende Aufnahmen darstellen, ist es vorteilhaft, wenn das Bildaufnahmemittel eine entsprechende Einrichtung, beispielsweise einen Laserscanner, aufweist, mit der ein solcher Oberflächenscan über das Körperteil durchführt werden kann. Eine solche Einrichtung ist so auszulegen, dass sie die Scanns mit hoher Geschwindigkeit erstellt, so dass eine schnelle Abfolge vieler Einzelbilder möglich ist.

In einer einfacheren Ausführungsform bedient sich das System einer Kamera, wobei unterschiedliche Gestaltungen möglich sind. Zum einen kann das Bildaufnahmemittel eine im Instrumentenkopf endende Licht leitende Faser sein, an deren anderen Ende sich die Kamera befindet. Zum anderen kann die Kamera im Instrument selber angeordnet werden, um die Oberfläche in der Umgebung des Instruments zu erfassen. Generell ist es jedoch vorteilhaft, wenn mittels einer im Instrumentenkopf Licht leitenden Faser Licht an das Behandlungsfeld geführt wird. Damit wird zum einem die Qualität der Aufnahmen deutlich verbessert. Zudem erleichtert das zusätzliche Licht dem Operateur die Orientierung. In einer besonders vorteilhaften Ausführung weist das Bildaufnahmemittel eine auf das Körperteil automatisch fokussierende Linsenoptik auf.

Die Behandlungsplanung wurde im Vorfeld der Behandlung an einem Computer vorgenommen, in dem die Planung als Planungsdaten im System der Körperdaten hinterlegt werden. Dabei können die Körperdaten anhand von Schnittbildern erstellt werden, die von einem Computertomographen (CT), einem Kemspintomographen (MR) oder einem dezidierten dental 3D bildgebenden Gerät erzeugt sind. Im Falle einer Zahnbehandlung können im Rahmen der Planung die exakte Lage des Bohrkanals und dessen korrekte Tiefe vorbestimmt werden. Die Instrumentendaten werden als Ist-Daten dabei durch das Ortungsmittel erhoben. Das Koordinatensystem der Körperdaten (Körperkoordinatensystem, KOS) wird im Rahmen der Registrierung in Übereinstimmung gebracht mit dem Koordinatensystem der realen Behandlung (OP-KOS). Die Lage des Instrumentes wird durch das Ortungsmittel erfasst und virtuell in die Körperdaten enthaltend auch die Planungsdaten projiziert. Eine Visualisierung des Instrumentes im System der Körperdaten erfolgt in Relation zum Körper.

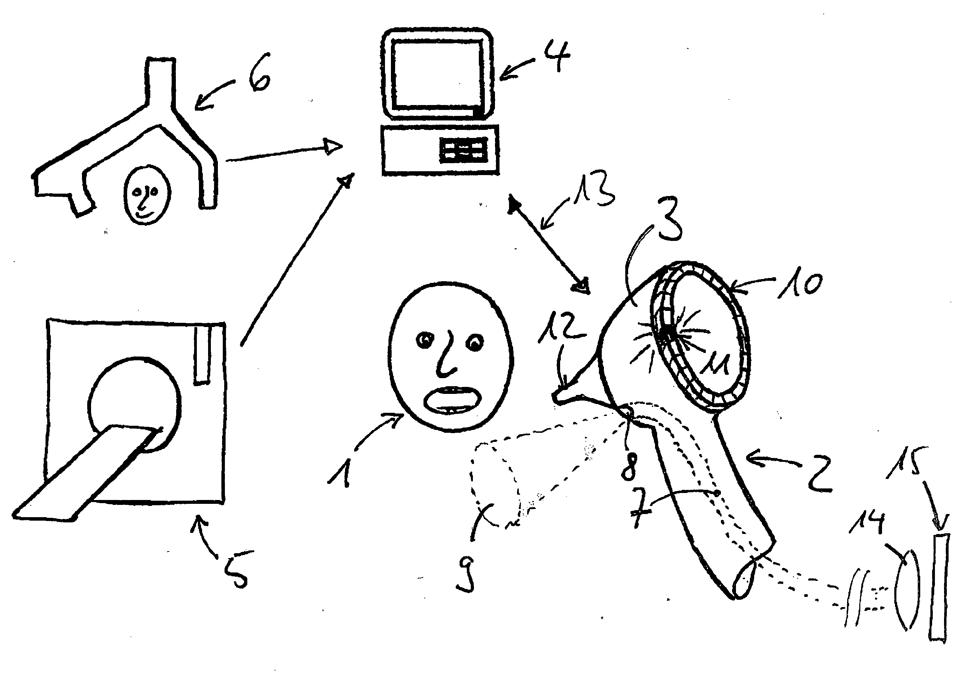

Ein Ausführungsbeispiel der Erfindung ist in der Figur dargestellt und wird nachfolgend näher beschrieben.

Die Figur zeigt ein System zur Durchführung einer Kiefer- oder Zahnbehandlung, die an einem Patienten 1 durchgeführt wird. Das System umfasst zunächst einen Dentalbohrer 2 als ein für den Arzt handhabbares Instrument mit einem den Kiefer beaufschlagenden und einen Bohrer tragenden Instrumentenkopf 3. Zudem umfasst das System eine Kontrolleinheit mit einem Computer 4, auf dem ein Navigationsprogramm zur Führung des Instrumentenkopfes 3 realisiert ist. Der Dentalbohrer 2 ist über eine Datenleitung 13 mit dem Computer 4 verbunden. Das Navigationsprogramm greift auf Körperdaten zu, die von einem CT 5 oder einem dental 3D bildgebenden Gerät 6 erzeugt sind und an denen die Planung durchgeführt wurde.

Im Vorfeld des Eingriffs werden mittels eines der Geräte 5 oder 6 Planungsdaten aufgenommen, die eine Grundlage für das Navigationsprogramm bilden. Die für die Behandlungsplanung benötigten Bilder können sowohl im Vorfeld aber auch während des Eingriffs generiert werden. Sie können mit bildgebenden Verfahren unterschiedlicher Techniken, wie Röntgen, Magnetresonanz Ultraschall und/oder optisch aufgenommen werden. Die Planung wird ebenfalls am Computer 4 durchgeführt. Während der Behandlung wird die Lage des Instrumentenkopfes innerhalb der Körperdaten bestimmt und mit den Planungsdaten verglichen. Zur Anzeige von Abweichungen ist am Dentalbohrer 2 ein Signalmittel 10 vorhanden, das dem Operateur Abweichungen der Instrumentendaten von den Planungsdaten anzeigt, wobei die Signale wahrnehmbar sind, während der Operateur den Instrumentenkopf unmittelbar in seinem Blickfeld hat.

Das Ortungsmittel weist einen Lichtleiter 7 auf, der in der Nähe des Instrumentenkopfes 12 in einem Auge 8 mündet. Mit diesem Auge 8 hat das Ortungsmittel ein Sichtfeld 9, in dem der Behandlungsbereich liegt. Der Lichtleiter 7 führt aus dem Dentalbohrer 2 hinaus zu einem Aufnahmemittel, das eine Linse 14 und eine elektronische Kamera 15 umfasst. Dort wird jedes Einzelbild digital erfasst und dem Navigationsprogramm übermittelt. Auf diese Weise werden die Instrumentendaten in einem den Raum der Körperdaten aufspannenden Koordinatensystem erfasst. Sowohl die Bildgebenden Geräte 5 und 6 als auch das Aufnahmemittel sind mit jeweils einer Datenleitung mit dem Computer 4 verbunden und übermitteln die Körperdaten respektive die Instrumentendaten über diese Leitungen.

Wie die Figur zeigt, besitzt der Bohrer am Bohrkopf LED's 10 als Signalmittel, die zu einem Kranz angeordnet sind. Über eine variierende Leuchtstärke der im Kranz angeordneten Leuchtmittel lässt sich ein Zeiger realisieren, der die Funktion einer in die richtige Richtung weisenden Kompassnadel hat. Die LED's 10 werden über den Rechner 4 angesteuert, der auch die zuvor abgespeicherten Planungsdaten erhält und der, wie beschrieben, in Verbindung mit dem Navigationssystem steht. Zuerst positioniert der Arzt die Spitze 12 des Bohrers 2, wobei er durch die rot blinkende LED 11 des Kranzes 10 in die Richtung gewiesen wird. Die zur Navigation nötige Information ermittelt der Rechner wie oben beschrieben aus den Planungsdaten und der Lage des Instruments, die er vom Navigationssystem erhält. Hat der Operateur die Position im Rahmen einer definierten Toleranz erreicht, leuchtet der gesamte äußere LED Kranz. Danach stellt er die korrekte Ausrichtung des Bohrers ein, indem er den Bohrer 2 in die Richtung neigt, die ihm jetzt durch eine blau leuchtende LED auf dem Kranz 10 visualisiert wird. Stimmt nun die Position der Spitze und die Lage im Raum, wechselt der gesamte äußere Kranz seine Farbe und leuchtet grün. Nun kann der Arzt bohren, da der Bohrer exakt entlang der Planung ausgerichtet ist. Hat er die korrekte Tiefe erreicht blinkt der Kranz. Zusätzlich kann vom Rechner auch noch ein akustisches Signal gegeben werden.

## Patentansprüche

1. System zur Durchführung einer Behandlung eines menschlichen oder tierischen Körpers durch einen Operateur, umfassend ein handhabbares Instrument (2) mit einem ein Operationsfeld des Körpers beaufschlagenden und ein Behandlungswerkzeug (12) tragenden Instrumentenkopf (3) und umfassend einen Computer (4), auf dem ein Navigationsprogramm zur Unterstützung der Führung des Instrumentenkopfes (3) realisiert ist, wobei dem Navigationsprogramm den das Operationsfeld beinhaltenden Teil des Körpers repräsentierende Körperdaten, die geplante Behandlung repräsentierende Planungsdaten und die Position und die Ausrichtung des Instrumentenkopfes (3) repräsentierende Instrumentendaten zur Verfügung stehen, wobei ein Ortungsmittel zur Aufnahme der Instrumentendaten vorhanden ist, wobei das Navigationsprogramm die Instrumentendaten als Ist-Daten mit den Planungsdaten als Soll-Daten vergleicht, wobei ein Signalmittel (10) vorgesehen ist, das dem Operateur eine Abweichung der Ist-Daten von den Soll-Daten anzeigt,
**dadurch gekennzeichnet,**
**dass** das Ortungsmittel ein am Instrumentenkopf (3) befindliches Bildaufnahmemittel aufweist, das während der Handhabung des Instrumentenkopfes Einzelbilder eines in den Körperdaten repräsentierten und mit der Behandlungsstelle in definiertem Bezug stehenden Körperteils insbesondere in schneller Abfolge, aufnimmt, wobei die Ausrichtung des Bildaufnahmemittels in definiertem Bezug zum Instrumentenkopf steht, wobei das Navigationsprogramm durch Abgleich der Einzelbilder mit den Körperdaten die Instrumentendaten erzeugt, und
**dass** das Navigationsprogramm zum Abgleich der Einzelbilder mit den Körperdaten massive Strukturen, nämlich freigelegte Knochen und/oder Zähne, aus den Körperdaten herausfiltert.

2. System nach dem vorherigen Anspruch,
**dadurch gekennzeichnet, dass** das Signalmittel (10) am Instrumentenkopf (3) vorgesehen ist, wobei das Signalmittel (10) dem Operateur die Abweichung der Ist-Daten von den Soll-Daten anzeigt, während er den Instrumentenkopf (3) im Blickfeld hat, wobei das Instrument insbesondere ein Dentalbohrer (2) ist.

3. System nach einem der vorherigen Ansprüche,
**dadurch gekennzeichnet, dass** das Ortungsmittel zwei Bildaufnahmemittel aufweist, die eine zur Erzeugung einer stereoskopischen Aufnahme des Körperteils geeignete Orientierung zueinander haben.

4. System nach einem der vorherigen Ansprüche,
**dadurch gekennzeichnet, dass** das Blickfeld (9) des Bildaufnahmemittels auf das Operationsfeld und insbesondere auch auf den Instrumentenkopf, respektive das Behandlungswerkzeug (12) gerichtet ist.

5. System nach Anspruch 4,
**dadurch gekennzeichnet, dass** das aktuelle Bild des Bildaufnahmemittels auf einem für den Operateur einsehbaren Bildschirm angezeigt wird.

6. System nach einem der vorherigen Ansprüche,
**dadurch gekennzeichnet, dass** das Bildaufnahmemittel eine Einrichtung aufweist, die Oberflächenscans über das Körperteil durchführt und daraus die Einzelbilder erzeugt.

7. System nach einem der vorherigen Ansprüche,
**dadurch gekennzeichnet, dass** das Bildaufnahmemittel eine im Instrumentenkopf endende lichtleitenden Faser (7) ist, an deren anderen Ende sich eine Kamera (15) befindet.

8. System nach einem der vorherigen Ansprüche,
**dadurch gekennzeichnet, dass** mittels einer im Instrumentenkopf endenden lichtleitenden Faser (7) Licht an das Operationsfeld führbar ist.

9. System nach einem der vorherigen Ansprüche,
**dadurch gekennzeichnet, dass** das Bildaufnahmemittel eine auf das Körperteil automatisch fokussierende Linsenoptik (14) aufweist.

## Claims

1. A system for carrying out a treatment of a human or animal body by a surgeon, comprising a hand-held instrument (2) having an instrument head (3) that acts on an operating field of the body and supports a treatment tool (12), and comprising a computer (4) on which a navigation program is provided to assist the guidance of the instrument head (3), wherein body data representing the part of the body containing the operating field, planning data representing the planned treatment, and instrument data representing the position and orientation of the instrument head (3) are available to the navigation program, wherein a positioning means for recording the instrument data is present, wherein the navigation program compares the instrument data as actual data with the planning data as desired data, wherein a signalling means (10) is provided, which indicates to the surgeon a deviation of the actual data from the desired data,
**characterised in that**
the positioning means comprises an image-recording means that is located at the instrument head (3) and that records, during the handling of the instrument head and in particular in rapid sequence, single images of a body part that is represented in the body data and is in a defined relationship with the treatment site, wherein the orientation of the image-recording means is in a defined relationship with the instrument head, wherein the navigation program generates the instrument data by matching the single images with the body data, and
that the navigation program, for comparison of the single images with the body data, filters out solid structures from the body data, such as exposed bone and/or teeth.

2. The system according to the preceding claim,
**characterised in that** the signalling means (10) is provided on the instrument head (3), wherein the signalling means (10) indicates to the surgeon the deviation of the actual data from the desired data whilst the instrument head (3) is in his field of vision, wherein the instrument is a dental drill (2) in particular.

3. The system according to any of the preceding claims,
**characterised in that** the positioning means has two image-recording means, which are oriented relative to one another in a manner suitable for generating a stereoscopic recording of the body part.

4. The system according to any of the preceding claims,
**characterised in that** the field of vision (9) of the image-recording means is directed to the operating field and in particular also to the instrument head or the treatment tool (12).

5. The system according to claim 4,
**characterised in that** the current image of the image-recording means is displayed on a screen visible to the surgeon.

6. The system according to any of the preceding claims,
**characterised in that** the image-recording means has a device that carries out surface scans over the body part and generates the single images therefrom.

7. The system according to any of the preceding claims,
**characterised in that** the image-recording means is a light-conducting fibre (7) ending in the instrument head, at the other end of which a camera (15) is located.

8. The system according to any of the preceding claims, **characterised in that** light can be guided to the operating field by means of a light-conducting fibre (7) ending in the instrument head.

9. The system according to any of the preceding claims, **characterised in that** the image-recording means has a lens optic (14) focussing automatically on the body part.

## Revendications

1. Système de réalisation d'un traitement d'un corps humain ou animal par un opérateur, comprenant un instrument maniable (2) doté d'une tête d'instrument (3) sollicitant un champ d'opération du corps et portant un outil de traitement (12) et comprenant un ordinateur (4) sur lequel est exécuté un programme de navigation pour assister le guidage de la tête d'instrument (3), des données corporelles représentant le champ d'opération, des données d'organisation représentant le traitement planifié et des données d'instrument représentant la position et l'orientation de la tête d'instrument (3) étant à la disposition du programme de navigation, un moyen de localisation destiné à recevoir les données d'instrument étant prévu, le programme de navigation comparant les données d'instrument sous forme de données réelles aux données d'organisation sous forme de données théoriques, étant prévu un moyen de signalisation (10) qui affiche à l'opérateur un écart entre les données réelles et les données théoriques,
**caractérisé en ce que**
le moyen de localisation présente un moyen de prise de photos se trouvant à la tête de l'instrument (3) et qui, pendant le maniement de la tête d'instrument, prend des photos individuelles d'une partie du corps représentant les données corporelles et étant en relation définie avec le point de traitement, en particulier en succession rapide, l'orientation du moyen de prise de photos étant en relation définie avec la tête d'instrument, le programme de navigation générant les données d'instrument en alignant les photos individuelles sur les données corporelles, et
que le programme de navigation, pour aligner les photos individuelles sur les données corporelles, expurge les structures massives, à savoir les os et/ou dents découverts, des données corporelles.

2. Système selon la revendication précédente,
**caractérisé en ce que**
le moyen de signalisation (10) est prévu au niveau de la tête d'instrument (3), le moyen de signalisation (10) affichant à l'opérateur l'écart entre les données réelles et les données théoriques pendant qu'il a la tête d'instrument (3) dans son champ visuel, l'instrument étant en particulier une fraise dentaire (2).

3. Système selon une des revendications précédentes,
**caractérisé en ce que**
le moyen de localisation présente deux moyens de prise de photos qui ont entre eux une orientation appropriée à la génération d'un cliché stéréoscopique de la partie du corps.

4. Système selon une des revendications précédentes,
**caractérisé en ce que**
le champ visuel (9) du moyen de prise de photos est dirigé sur le champ d'opération et en particulier sur la tête d'instrument ou l'outil de traitement (12).

5. Système selon la revendication 4,
**caractérisé en ce que**
l'image actuelle du moyen de prise de photos est affichée sur un écran visible par l'opérateur.

6. Système selon une des revendications précédentes,
**caractérisé en ce que**
le moyen de prise de photos présente un dispositif qui réalise des balayages de surface au-dessus de la partie du corps et génère les photos individuelles à partir de ceux-ci.

7. Système selon une des revendications précédentes,
**caractérisé en ce que**
le moyen de prise de photos est une fibre optique (7) se terminant dans la tête d'instrument et à l'autre extrémité de laquelle se trouve une caméra (15).

8. Système selon une des revendications précédentes,
**caractérisé en ce que**
de la lumière peut être conduite vers le champ d'opération au moyen d'une fibre optique (7) se terminant dans la tête d'instrument.

9. Système selon une des revendications précédentes,
**caractérisé en ce que**
le moyen de prise de photos présente une optique à lentilles (14) se focalisant automatiquement sur la partie du corps.
